(19) Europäisches Patentamt
European Patent Office
Office européen des brevets

(11) **EP 3 396 373 A1**

(12) **DEMANDE DE BREVET EUROPEEN**

(43) Date de publication:
**31.10.2018 Bulletin 2018/44**

(51) Int Cl.:
***G01N 33/20*** (2006.01)

(21) Numéro de dépôt: **18169511.5**

(22) Date de dépôt: **26.04.2018**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **26.04.2017 FR 1753633**

(71) Demandeur: **ANKEN**
**31500 Toulouse (FR)**

(72) Inventeur: **BOURGE, Michel**
**31500 TOULOUSE (FR)**

(74) Mandataire: **Cabinet Plasseraud**
**66, rue de la Chaussée d'Antin**
**75440 Paris Cedex 09 (FR)**

(54) **DISPOSITIF DE MESURE ET PROCÉDÉ DE CARACTÉRISATION POUR L'ACIÉRIE ÉLECTRIQUE**

(57)   L'invention concerne un dispositif de mesure (100) destiné à être branché sur un réseau d'alimentation électrique (200) d'un four à arc électrique (300) contenant des matières solides ou fondues de métal en fusion, comme de l'acier liquide. Le dispositif comprend :
- au moins un capteur (110) pour mesurer au moins un signal électrique du réseau d'alimentation électrique, pendant une période de temps prédéterminée,
- un spectromètre (120) pour générer un spectre du signal électrique mesuré,
- un processeur (130) pour traiter le spectre généré, en utilisant un modèle multivariable prédictif qui définit une fonction de transfert pour chacun parmi au moins deux caractéristiques chimiques et/ou physiques des matières et le spectre du signal électrique associé à l'alimentation du four à arc électrique, pour déterminer au moins une caractéristique chimique et/ou physique.

FIG. 1

EP 3 396 373 A1

**Description**

Domaine technique

La présente invention concerne un dispositif de mesure et un procédé de détermination d'au moins une caractéristique chimique et/ou physique de matières solides ou fondues de métal en fusion, comme de l'acier liquide, se trouvant à l'intérieur d'un four à arc électrique.

Technique antérieure

Dans le domaine de l'aciérie électrique, les fondeurs utilisent essentiellement le four à arc électrique comme outil de production de métal liquide, notamment pour l'acier, les ferro-alliages, le manganèse, le chrome, le plomb ou encore le silicium. En effet, un tel four est destiné à fondre par fusion un métal primaire, tel que de la ferraille de récupération, de la fonte (solide ou liquide) ou encore des minerais préréduits.

Le four à arc électrique est un système électrothermique utilisant l'énergie fournie par un arc électrique pour chauffer une charge. En effet, le principe du four à arc électrique consiste à faire éclater un arc électrique entre une électrode en graphite qui amène le courant électrique et la charge métallique à fondre qui sert de masse. Ensuite, la chaleur dégagée par l'arc électrique est directement utilisée pour la fusion.

Afin d'obtenir les caractéristiques demandées au métal par le cahier des charges, les fondeurs doivent maîtriser la composition chimique du métal liquide et la température de coulée. La composition chimique du métal liquide est généralement requise par le client et/ou les normes applicables. La température de coulée est déterminante pour assurer le remplissage des moules, la santé interne des pièces et également pour obtenir des conditions de solidification et une structure métallographique correcte.

En pratique, les fondeurs utilisent des sondes pyrométriques qui sont introduites dans le métal liquide afin de mesurer la température du métal liquide et également extraire des échantillons aux fins d'analyses chimiques.

Cette opération délivre une information ponctuelle dans le temps et nécessite d'être renouvelée plusieurs fois durant un cycle d'élaboration en vue d'arriver au plus près des caractéristiques recherchées. Or, cette opération peut être réalisée manuellement, ce qui présente un risque pour les personnes la réalisant. Cette opération peut également être réalisée au moyen d'un robot manipulateur, ce qui peut être couteux.

Concernant l'obtention de la composition chimique, l'appareil de mesure ne se trouve pas généralement à proximité immédiate du four. Ceci a pour effet que le temps nécessaire d'analyse peut varier énormément de sorte que le cycle de fonctionnement du four doit être suspendu, dans l'attente des résultats. En effet, le temps d'analyse dépend, entre autres, du trajet que doit suivre l'échantillon jusqu'au laboratoire, du type d'analyse à réaliser, ou encore de la longueur de la file d'attente au niveau du laboratoire d'analyses dans la mesure où un laboratoire peut réaliser des analyses pour des outils d'élaboration différents.

Résumé de l'invention

La présente invention vise donc à pallier de tels inconvénients en procurant un dispositif de mesure en continu ainsi qu'un procédé permettant de déterminer au moins une caractéristique chimique et/ou physique de matières solides ou fondues de métal en fusion, comme de l'acier liquide, se trouvant à l'intérieur d'un four à arc électrique, et ce, sans contact direct avec lesdites matières.

Des caractéristiques de la présente invention sont définies dans les revendications ci-jointes.

Brève description des dessins

D'autres caractéristiques et avantages de l'invention seront mieux compris à la lecture de la description qui va suivre et en référence aux dessins annexés, donnés à titre illustratif et nullement limitatif.

La figure 1 représente une vue fonctionnelle schématique d'un dispositif de mesure selon une mise en oeuvre de l'invention.
La figure 2 représente une représentation graphique d'un spectre.
La figure 3 représente un organigramme selon une mise en oeuvre de l'invention.

Pour des raisons de clarté, les éléments représentés ne sont pas à l'échelle les uns par rapport aux autres, sauf mention contraire.

Description des modes de réalisation

Avant de décrire l'invention, il semble utile de brièvement rappeler le principe de fonctionnement d'un four à arc électrique. Un tel four se compose d'une cuve garnie de réfractaires et l'énergie nécessaire à la fusion est fournie par au moins un arc électrique jaillissant entre des électrodes en graphites, raccordées à un réseau de puissance, et la charge métallique à chauffer. Dans le four à arc électrique, le métal fond par attaque directe de l'arc électrique qui creuse des puits dans la charge métallique. Lorsque ces puits se sont formés, l'arc travaille sur un bain liquide dont le volume augmente au cours de la fusion. La fusion s'opère par couches successives de l'intérieur vers l'extérieur de la masse des matières premières chargées dans le four. Lorsque la technique du laitier moussant (injection simultanée de carbone et d'oxygène) est mise en oeuvre, la puissance maximale de l'arc électrique peut être utilisée pendant pratiquement toute l'élaboration car l'arc électrique est noyé dans le laitier. La capacité d'un four à arc électrique varie de 2 à 150 tonnes, pour une puissance de **1200** kVA (kilovoltampère) à 190 MVA (mégavoltampère). Enfin, le cycle de fonctionnement d'un four à arc électrique dure entre 40 minutes et 4 heures.

Le principe général de l'invention est basé sur l'instabilité de l'arc électrique qui est connu pour être due à sa non-linéarité. Il a été remarqué que la non-linéarité de l'arc électrique produit des fluctuations sous formes d'harmoniques, tout au long de l'élaboration du métal liquide, au niveau de l'alimentation du four à arc électrique. Il est à noter que les phénomènes physiques mis en jeu dans ces fluctuations sont complexes et ne sont pas encore suffisamment compris pour permettre une modélisation complète du processus. L'intuition originale selon la présente invention a consisté à considérer que la charge présente dans un four à arc électrique est également une source non-linéaire produisant des harmoniques au niveau de l'alimentation du four à arc électrique. Il a donc été supposé qu'il existe une corrélation entre les caractéristiques chimiques et/ou physiques des phases condensées présentes dans un four à arc électrique et les harmoniques produites dans l'alimentation du four à arc électrique. Des expérimentations ont permis de déterminer des fonctions de transfert pour chaque caractéristique chimique et/ou physique du bain métallique ainsi que du laitier. Par exemple des caractéristiques chimiques du laitier comme sa basicité, ou encore des caractéristiques du bain métallique, telles que la teneur en carbone, en manganèse, en silice ou en oxyde de calcium peuvent être déterminées à partir des fonctions de transfert obtenues. Il en est de même pour des caractéristiques physiques du bain, telle que la température. L'identification des fonctions de transfert est basée sur l'utilisation de méthodes statistiques d'analyse dites multivariées. Enfin, l'ensemble des fonctions de transfert est utilisé dans un modèle multivariable prédictif afin de déterminer, à un moment donné, la composition chimique ainsi que la température des phases condensées à partir d'un spectre courant du signal d'alimentation du four à arc électrique.

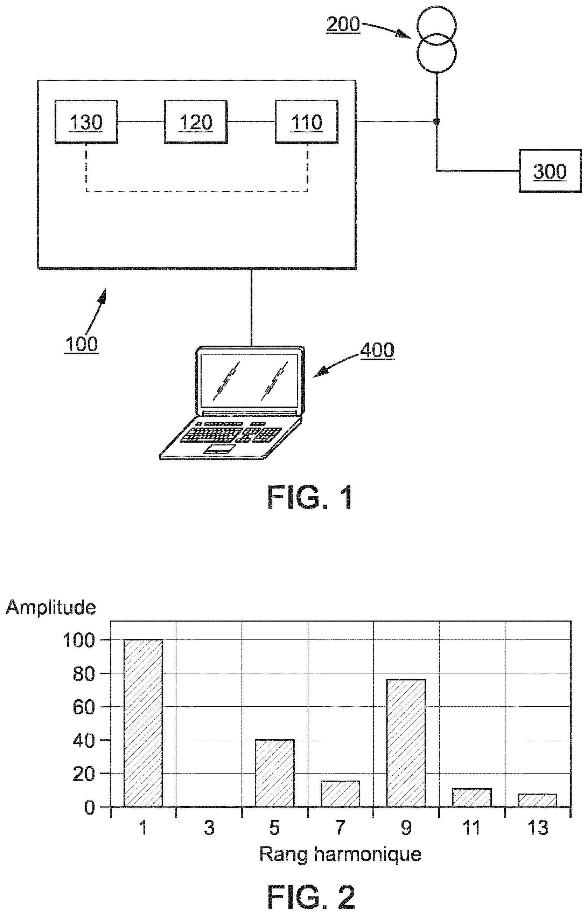

L'exemple de la figure 1 montre un dispositif de mesure **100** destiné à être branché sur un réseau d'alimentation électrique **200** d'un four à arc électrique **300** contenant des matières (non représentées) solides ou fondues de métal en fusion, comme de l'acier liquide. Dans l'exemple de la figure 1, le réseau d'alimentation **200** est un transformateur de four, du type communément utilisé dans le domaine de l'aciérie, tel un transformateur colonne ou un transformateur cuirassé. Le réseau d'alimentation électrique **200** est configuré pour délivrer un courant alternatif ou continu. Toutefois, dans le reste de la description, on considèrera que le réseau d'alimentation électrique délivre un courant alternatif.

Dans la figure 1, le dispositif de mesure **100** comprend un capteur **110,** un spectromètre **120** et un processeur **130.** Le capteur **110** est couplé au spectromètre **120** et le spectromètre est couplé au processeur **130.**

Le capteur **110** de la figure 1 est configuré pour mesurer au moins un signal électrique du réseau d'alimentation électrique **200.** Par exemple, une acquisition d'un signal électrique comprenant une plage d'amplitude d'au moins $\pm 5V$ semble suffisante pour obtenir des résultats fiables. Le capteur **110** comprend un convertisseur analogique-numérique (non représenté) pour présenter le signal électrique mesuré sous forme numérique. Dans une mise en oeuvre, le capteur **110** mesure un courant circulant dans le réseau d'alimentation électrique au niveau d'un des conducteurs du circuit secondaire d'alimentation du four **300.** Dans une autre mise en oeuvre, le capteur **110** mesure une tension présente sur le réseau d'alimentation électrique **200** au niveau d'un des conducteurs du circuit secondaire d'alimentation du four **300.** Par exemple, le capteur **110** est un capteur du type inductif ou un capteur exploitant l'effet Hall. En outre, dans une première mise en oeuvre, le capteur **110** comprend une horloge programmable (non représentée) qui est configurée pour définir une période de temps prédéterminée pendant laquelle la mesure est réalisée. Dans un exemple, une valeur de période de trois à dix secondes, fixée en usine, peut être envisagée. De si courtes périodes sont choisies à cause du fait que les phénomènes harmoniques liés à la non-linéarité de la charge du four **300** semblent varier rapidement. Dans une deuxième mise en oeuvre particulière, le capteur **110** est couplé au processeur **130,** et le processeur **130** est configuré pour définir la valeur de période prédéterminée en fonction des besoins. Dans une troisième mise en oeuvre particulière, le capteur **110** est couplé au processeur **130** mais le processeur **130** comprend l'horloge programmable. Dans cette mise en oeuvre, le processeur **130** est configuré pour définir la valeur de période prédéterminée et commander le fonctionnement du capteur **110** en fonction de la période prédéterminée.

Le spectromètre **120** de la figure 1 est configuré pour générer un spectre de fréquences du signal électrique mesuré par le capteur **110.** Le spectre ainsi généré comprend alors une pluralité de raies spectrales présentant chacune une amplitude et une phase propres. Dans une mise en oeuvre, le spectromètre **120** génère le spectre de fréquences en appliquant une transformée de Fourier discrète au signal électrique mesuré. Lorsque plusieurs spectres sont générés pendant une période prédéterminée, le spectromètre **120** peut réaliser une moyenne de l'ensemble des amplitudes et phases des harmoniques associées à chaque spectre généré.

La figure 2, montre un exemple de spectre obtenu en appliquant une transformée de Fourier discrète à un signal électrique mesuré par le capteur **110.** Il est connu qu'une telle opération permet de quantifier la distorsion harmonique d'un signal. Ainsi, par exemple, un signal périodique possédant une fréquence fondamentale $f_0$ peut être décomposé en une somme de signaux sinusoïdaux dont chacun possède une fréquence, dite harmonique, qui est un multiple entier (appelé rang harmonique) de la fréquence fondamentale f$_0$. L'exemple de la figure 2 illustre en son abscisse, les rangs harmoniques impairs d'un signal donné. Toutefois, il est également possible de représenter les rangs harmoniques pairs pour un signal donné. En ordonnée de la figure 2, est exprimée l'amplitude de chaque composante harmonique, en pourcentage de la grandeur fondamentale correspondante, appelée taux d'harmoniques.

Le processeur **130** de la figure 1 est configuré pour traiter le spectre généré par le spectromètre **120** pour déterminer au moins une caractéristique chimique et/ou physique des matières présentes dans le four **300**. Pour cela, le processeur **130** utilise un modèle multivariable prédictif qui définit une fonction de transfert pour chacun parmi au moins deux caractéristiques chimiques et/ou physiques des matières et le spectre du signal électrique associé à l'alimentation du four à arc électrique **300**.

Dans une mise en oeuvre, le modèle multivariable prédictif est obtenu préalablement, au moyen d'une méthode statistique d'analyse multivariée. Par exemple, on pourra utiliser une régression sur composantes principales (en anglais, PCR pour Principal Component Regression), une régression par les moindres carrés partiels (en anglais, PLS pour Partial Least-Squares) ou encore les méthodes d'intelligence artificielle telles que les réseaux de neurones ou le machine learning. Le modèle multivariable prédictif permet d'estimer la composition chimique/physique des matières présentes dans le four **300** à partir du spectre d'une mesure d'un signal électrique du réseau d'alimentation électrique **200** du four **300**. Le modèle multivariable prédictif peut être représenté sous forme mathématique comme suit :

$$p_k = f_k(s) \ \ (1),$$

où

- $p_k$ est la caractéristique chimique et/ou physique des matières présentes dans le four **300** dont on recherche la concentration à un moment donné,
- $f_k(\cdot)$ est la fonction de transfert associée à ladite caractéristique chimique et/ou physique, et
- $s$ est le spectre d'une mesure d'un signal électrique du réseau d'alimentation électrique **200** du four **300**.

Enfin, le processeur **130** de la figure 1 est en outre configuré pour normaliser le spectre généré par rapport au maximum d'amplitude du spectre généré. Ceci a pour effet de rendre le modèle indépendant de la fréquence fondamentale du spectre de l'alimentation du four **300**. Ensuite, le processeur **130** est également configuré pour déterminer la au moins une caractéristique chimique et/ou physique à partir d'une somme pondérée du spectre normalisé en fonction des coefficients de régression de la fonction de transfert correspondante. Toutefois, il est également envisagé que le processeur réalise une telle somme pondérée du spectre généré à la place du spectre normalisé.

Dans la figure 1, le dispositif de mesure **100** est couplé à un dispositif d'affichage **400** tel qu'un écran d'ordinateur, de smartphone, de tablette, de télévision, une enseigne, un panneau indicateur ou encore un tableau indicateur. Le dispositif d'affichage **400** est configuré pour afficher la au moins une caractéristique chimique et/ou physique.

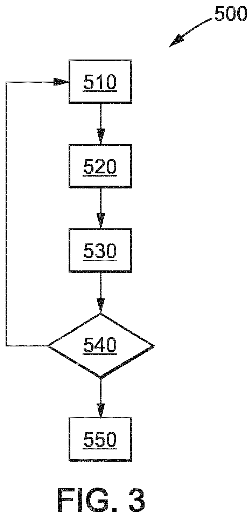

La figure 3 illustre un exemple de procédé **500** d'obtention du modèle multivariable prédictif.

Tout d'abord dans une étape **510,** on réalise simultanément un prélèvement d'un échantillon de matières du four **300** et on mesure un signal électrique du réseau d'alimentation électrique du four **300**. Comme indiqué plus haut, le prélèvement peut être réalisé à partir de sondes pyrométriques et la mesure peut être réalisée avec le capeur **110.**

Ensuite, dans l'étape **520**, on détermine une composition chimique et/ou physique de l'échantillon de matières prélevé, la composition chimique et/ou physique comprenant des concentrations d'au moins deux caractéristiques chimiques et/ou physiques. Par exemple, on peut faire appel à des techniques de spectrométrie d'émission optique (en anglais, OES pour Optical Emission Spectrometry) pour obtenir la composition chimique tandis qu'une sonde pyrométrique fournira la température de l'échantillon.

Dans l'étape **530**, on génère un spectre du signal électrique mesuré. Comme indiqué plus haut, la génération du spectre peut être réalisée au moyen du spectromètre **120**.

Par la suite, à l'étape **540**, on répète plusieurs fois les étapes **510, 520** et **530** pendant au moins une partie de la durée d'au moins un cycle de fonctionnement du four **300**. Dans un exemple, l'ensemble des données de mesures spectrométriques et l'ensemble des compositions chimiques et/ou physiques des échantillons prélevés sont regroupées dans une matrice X, comprenant n lignes et m colonnes, avec n le nombre de mesures spectrométriques et m le nombre de compositions chimiques et/ou physiques des échantillons prélevés.

Enfin, à l'étape **550**, on génère le modèle multivariable prédictif, au moyen d'une méthode statistique d'analyse multivariée, en obtenant des coefficients de régression d'une fonction de transfert pour chacun parmi au moins deux caractéristiques chimiques et/ou physiques. Dans un exemple, on applique à la matrice X, une régression sur composantes principales ou une régression par les moindres carrés partiels. On obtient alors une estimation de coefficients de régressions a$_1$, a$_2$, ..., a$_k$ pour chacun des éléments des compositions chimiques et/ou physiques des échantillons prélevés.

Le modèle multivariable prédictif peut alors être représenté sous forme matricielle comme suit :

$$\hat{y} = H \times c \ \ (2)$$

où,

- $\hat{y}$ est le vecteur d'estimation de la composition chimique et/ou physique de matières associé à un spectre donné,
- $H$ est la matrice comprenant le spectre à étudier, et
- $c$ est le vecteur comprenant les coefficients de régression estimés $a_1$, $a_2$, ..., $a_k$.

L'équation (2) peut également être reformulée comme suit :

$$\hat{y}_k = \sum_{i=1}^{n} \left( a_i^k \times H_i \right) \ (3)$$

où,

- $k$ correspond à la caractéristique chimique et/ou physique mesurée,
- $i$ est un entier positif égal au nombre d'harmoniques associées à la caractéristique à $k$.

Dans une mise en oeuvre particulière, on normalise $H_i$ par rapport au maximum d'amplitude de $H_i$. En général, un tel maximum se situe au niveau de l'harmonique associée à la fréquence fondamentale du signal traité. Un tel agencement peut être formulé comme suit :

$$\hat{y}_k = \sum_{i=1}^{n} \left( a_i^k \times \frac{H_i}{H_0} \right) \ (4),$$

où

- $H_0$ correspond au maximum d'amplitude de $H_i$.

Il est également envisagé de revendiquer un procédé de détermination d'au moins une caractéristique chimique et/ou physique de matières solides ou fondues de métal en fusion, comme de l'acier liquide, se trouvant à l'intérieur du four **300**.

Un tel procédé de détermination comprend les étapes suivantes consistant à :

- mesurer au moins un signal électrique du réseau d'alimentation électrique, pendant une période de temps prédéterminée,
- générer un spectre du signal électrique mesuré,
- traiter le spectre généré, en utilisant un modèle multivariable prédictif tel que décrit plus haut, pour déterminer la au moins une caractéristique chimique et/ou physique.

Une mise en oeuvre particulière du procédé de détermination, comprend en outre, les étapes consistant à :

- normaliser le spectre généré par rapport au maximum d'amplitude du spectre généré ; et

- déterminer la au moins une caractéristique chimique et/ou physique à partir d'une somme pondérée du spectre normalisé en fonction des coefficients de régression de la fonction de transfert correspondante.

Dans une autre mise en oeuvre particulière du procédé de détermination, l'étape de mesure consiste à mesurer un courant et/ou une tension du réseau d'alimentation électrique.

Enfin, dans une autre mise en oeuvre du procédé de détermination, on établit préalablement le modèle multivariable prédictif au moyen d'une méthode statistique d'analyse multivariée choisie parmi : une régression sur composantes principales et une régression par les moindres carrés partiels.

L'invention permet de réaliser des mesures sans contact entre le dispositif de mesure et les matières comprises dans un four à arc électrique. Ceci a pour effet, que l'opération de supervision de l'élaboration d'un métal liquide ne représente plus de risque pour les personnes qui le réalisent. En outre, l'invention permet d'obtenir des informations de composition chimique et/ou physique en temps réel et de manière très rapide.

Dans les revendications, le terme "comporter" n'exclut pas d'autres éléments ou d'autres étapes. Les différentes caractéristiques présentées et/ou revendiquées peuvent être avantageusement combinées. Leur présence dans la description ou dans des revendications dépendantes différentes n'exclut pas cette possibilité. Enfin, les signes de référence aux dessins ne sauraient être compris comme limitant la portée de l'invention.

**Revendications**

1. Dispositif de mesure (100) destiné à être branché sur un réseau d'alimentation électrique (200) d'un four à arc électrique (300) contenant des matières fondues de métal en fusion, comme de l'acier liquide, le dispositif comprenant :

   - au moins un capteur (110) pour mesurer au moins un signal électrique du réseau d'alimentation électrique, pendant une période de temps prédéterminée,
   - un spectromètre (120) pour générer un spectre du signal électrique mesuré,
   - un processeur (130) pour traiter le spectre généré, en utilisant un modèle multivariable prédictif qui définit une fonction de transfert pour chacun parmi au moins deux caractéristiques chimiques et/ou physiques des matières et le spectre du signal électrique associé à l'alimentation du four à arc électrique, pour déterminer au moins une caractéristique chimique et/ou physique.

2. Dispositif de mesure selon la revendication 1, dans lequel le processeur est en outre configuré pour :

- normaliser le spectre généré par rapport au maximum d'amplitude du spectre de généré ; et
- déterminer la au moins une caractéristique chimique et/ou physique à partir d'une somme pondérée du spectre normalisé en fonction des coefficients de régression de la fonction de transfert correspondante.

3. Dispositif de mesure selon les revendications 1 à 2, dans lequel le capteur est en outre configuré pour mesurer un courant et/ou une tension du réseau d'alimentation électrique.

4. Dispositif de mesure selon les revendications 1 à 3, dans lequel le modèle multivariable prédictif est obtenu préalablement, au moyen d'une méthode statistique d'analyse multivariée choisie parmi : une régression sur composantes principales, une régression par les moindres carrés partiels ou encore les méthodes d'intelligence artificielle.

5. Dispositif de mesure selon les revendications 1 à 4, comprenant en outre, un moyen d'affichage (400) pour afficher la au moins une caractéristique chimique et/ou physique.

6. Procédé de détermination d'au moins une caractéristique chimique et/ou physique de matières fondues de métal en fusion, comme de l'acier liquide, se trouvant à l'intérieur d'un four à arc électrique, le four à arc électrique étant raccordé à un réseau d'alimentation électrique, le procédé comprenant les étapes consistant à :

- mesurer au moins un signal électrique du réseau d'alimentation électrique, pendant une période de temps prédéterminée,
- générer un spectre du signal électrique mesuré,
- traiter le spectre généré, en utilisant un modèle multivariable prédictif qui définit une fonction de transfert pour chacun parmi au moins deux caractéristiques chimiques et/ou physiques des matières et le spectre du signal électrique associé à l'alimentation du four à arc électrique, pour déterminer la au moins une caractéristique chimique et/ou physique.

7. Procédé selon la revendication 6, comprenant en outre, les étapes consistant à :

- normaliser le spectre généré par rapport au maximum d'amplitude du spectre généré ; et
- déterminer la au moins une caractéristique chimique et/ou physique à partir d'une somme pondérée du spectre normalisé en fonction des coefficients de régression de la fonction de transfert correspondante.

8. Procédé selon les revendications 6 à 7, dans lequel l'étape de mesure consiste à mesurer un courant et/ou une tension du réseau d'alimentation électrique.

9. Procédé selon l'une des revendications 6 à 8, dans lequel on établit préalablement le modèle multivariable prédictif au moyen d'une méthode statistique d'analyse multivariée choisie parmi : une régression sur composantes principales, une régression par les moindres carrés partiels ou encore les méthodes d'intelligence artificielle.

10. Procédé (500) selon la revendication 9 dans lequel l'établissement du modèle multivariable prédictif comprend les étapes consistant à :

- simultanément (510), prélever un échantillon de matières du four à arc électrique et mesurer un signal électrique du réseau électrique,
- déterminer (520) une composition chimique et/ou physique de l'échantillon de matières prélevé, la composition chimique et/ou physique comprenant des concentrations d'au moins deux caractéristiques chimiques et/ou physiques,
- générer (530) un spectre du signal électrique mesuré,
- répéter (540) plusieurs fois les étapes de prélèvement, de mesure, de détermination et de génération de spectre, pendant au moins une partie de la durée d'au moins un cycle de fonctionnement du four à arc électrique,
- générer (550) le modèle multivariable prédictif, au moyen de la méthode statistique d'analyse multivariée, en obtenant des coefficients de régression d'une fonction de transfert pour chacun parmi les au moins deux caractéristiques chimiques et/ou physiques.

FIG. 1

Rang harmonique

FIG. 2

500

510

520

530

540

550

FIG. 3

Europäisches
Patentamt

European
Patent Office

Office européen
des brevets

**RAPPORT DE RECHERCHE EUROPEENNE**

Numéro de la demande

EP 18 16 9511

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | VITO LOGAR ET AL: "Modeling and Validation of an Electric Arc Furnace: Part 1, Heat and Mass Transfer", ISIJ INTERNATIONAL, vol. 52, no. 3, 1 janvier 2012 (2012-01-01), pages 402-412, XP055422506, JP ISSN: 0915-1559, DOI: 10.2355/isijinternational.52.402 * section 1 et 2; figure 10; tableau 2 * | 1-10 | INV. G01N33/20 |
| X | & VITO LOGAR ET AL: "Modeling and Validation of an Electric Arc Furnace: Part 2, Thermo-chemistry", ISIJ INTERNATIONAL, vol. 52, no. 3, 1 janvier 2012 (2012-01-01), pages 413-423, XP055422508, JP ISSN: 0915-1559, DOI: 10.2355/isijinternational.52.413 * le document en entier * ----- | 1-10 | |
| | | | DOMAINES TECHNIQUES RECHERCHES (IPC) |
| | | | G01N |
| X | US 2011/292961 A1 (MATSCHULLAT THOMAS [DE] ET AL) 1 décembre 2011 (2011-12-01) * alinéa [0054] - alinéa [0063] * ----- | 1-10 | |
| X | TZANEV A T ET AL: "Modeling and simulation of EAF melt shop", AMERICAN CONTROL CONFERENCE, 2000. PROCEEDINGS OF THE 2000 JUNE 28-30, 2000, PISCATAWAY, NJ, USA,IEEE, vol. 3, 28 juin 2000 (2000-06-28), pages 2048-2052, XP010518268, ISBN: 978-0-7803-5519-4 * sections 1.1, 3.3, 3.5; figure 3 * ----- -/-- | 1-10 | |

Le présent rapport a été établi pour toutes les revendications

| Lieu de la recherche | Date d'achèvement de la recherche | Examinateur |
|---|---|---|
| La Haye | 6 juin 2018 | Martin, Hazel |

Europäisches
Patentamt

European
Patent Office

Office européen
des brevets

**RAPPORT DE RECHERCHE EUROPEENNE**

Numéro de la demande

EP 18 16 9511

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | RICHARD D. M. MACROSTY ET AL: "Dynamic Modeling of an Industrial Electric Arc Furnace", INDUSTRIAL & ENGINEERING CHEMISTRY RESEARCH., vol. 44, no. 21, 1 octobre 2005 (2005-10-01), pages 8067-8083, XP055422410, US ISSN: 0888-5885, DOI: 10.1021/ie050101b * Chapitres 1 et 4 * ----- | 1-10 | |
| X | OPITZ FLORIAN ET AL: "Physics-Based Modeling of Electric Operation, Heat Transfer, and Scrap Melting in an AC Electric Arc Furnace", METALLURGICAL AND MATERIALS TRANSACTIONS B, SPRINGER NEW YORK LLC, US, vol. 47, no. 2, 21 janvier 2016 (2016-01-21), pages 1489-1503, XP035944638, ISSN: 1073-5615, DOI: 10.1007/S11663-015-0573-X [extrait le 2016-01-21] * chapitre I; section E du chapitre V et section C du chapitre VIII; figures 9, 13 * ----- | 1-10 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| La Haye | 6 juin 2018 | Martin, Hazel |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

    .......................................................................
& : membre de la même famille, document correspondant

EPO FORM 1503 03.82 (P04C02)

page 2 de 2

## ANNEXE AU RAPPORT DE RECHERCHE EUROPEENNE
## RELATIF A LA DEMANDE DE BREVET EUROPEEN NO.

EP 18 16 9511

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

06-06-2018

| Document brevet cité au rapport de recherche | Date de publication | Membre(s) de la famille de brevet(s) | Date de publication |
|---|---|---|---|
| US 2011292961 A1 | 01-12-2011 | BR PI0924368 B1 | 27-03-2018 |
| | | CA 2751198 A1 | 12-08-2010 |
| | | CN 102308173 A | 04-01-2012 |
| | | EP 2394124 A1 | 14-12-2011 |
| | | JP 2012516938 A | 26-07-2012 |
| | | KR 20110126603 A | 23-11-2011 |
| | | RU 2011133683 A | 10-03-2013 |
| | | UA 103510 C2 | 25-10-2013 |
| | | US 2011292961 A1 | 01-12-2011 |
| | | WO 2010088972 A1 | 12-08-2010 |

EPO FORM P0460

Pour tout renseignement concernant cette annexe : voir Journal Officiel de l'Office européen des brevets, No.12/82